# EUROPEAN PATENT APPLICATION

(11) **EP 0 676 189 A2**
(43) Date of publication of application: **11.10.1995**
(21) Application number: 95250043.7
(22) Date of filing: 23.02.1995
(51) Int. Cl.: A61K 7/06, A61K 7/08

(54) **Mousse-forming compositions upon discharge from air powered, non-aerosol pump and spray dispensers, and articles comprising combinations thereof**

(30) Priority: 11.03.1994 US 212416
(71) Applicant: Colgate-Palmolive Company, New York, N.Y. 10022-7499 (US)
(72) Inventor: de Bondt, Veerle, B-5170 Profondeville (BE); Correia, Marthino, Wetherby LS 22 5RB (GB)
(74) Representative: UEXKÜLL & STOLBERG

(57) **Abstract**

A single phase aqueous composition which is to be discharged from an air-powered, non-aerosol, propellant-free, pump and spray dispenser to form a mousse, which composition comprises a clear solution of a monocationic quaternary surfactant salt, a hair holding polymeric material, and the remainder water, as well as the article comprising said dispenser and said composition. Optional ingredients may include a polycationic polymer, a UV protectant or sunscreen agent, and a water soluble or miscible hair conditioning agent and the article comprising said composition and said dispenser.

## Description

### BACKGROUND OF THE INVENTION

### Field of the Invention

The present invention relates to novel articles comprising the combination of hair styling (and, optionally, conditioning, voluminizing, and UV protecting) compositions packaged in environmentally safe, non-aerosol, propellant-free, air powered, "pump and spray" dispensers. More particularly, the present invention relates to aqueous compositions that form stable yet collapsible foams or mousses upon discharge from such dispensers. Even more particularly, the present invention relates to a single phase, aqueous composition which is to be discharged from a air-powered, non-aerosol pump and spray dispenser to form a mousse, which composition comprises a clear solution of a monocationic quaternary salt, a hair holding polymeric material, and the remainder water. Optional ingredients include a polycationic polymer; a water soluble or miscible hair conditioning agent; a voluminizing agent; a UV protectant and the like.

### The Prior Art

Conventional hair styling foam-forming, mousse compositions are generally multiphase in nature and are designed to be dispensed from aerosol containers which employ various water-insoluble propellants including hydrocarbon (propane, isobutane) and halohydrocarbon (Freon) gases or various water-soluble propellants (nitrous oxide, carbon dioxide, nitrogen). Because aerosol propellants have, at room temperature, a vapor pressure greater than one atmosphere but low enough to permit safe storage at room temperature in the liquid state under the pressures of a few atmospheres, they must be used in conventional valved aerosol containers. The propellant is sufficiently volatile so that upon the actuation of the valve and the resultant pressure release, it will quickly vaporize and leave the aerosol container. As is well known, the halohydrocarbon propellants are no longer in use due to their effect on the environment, the amount of other propellants in aerosol products must be limited in certain states in the U.S., and metal (aluminum) aerosol cans are also in disfavor due to environmental considerations in many countries of the world. Hence, there is a need for a product conventionally packaged as an aerosol in a metal can to be available in an environmentally friendly non-aerosol form. There is a particular problem with respect to the packaging of mousse products in non-aerosol forms, because mousses, which are in liquid, unfoamed state in the dispenser, must be capable of forming stable but relatively quick-breaking foams upon discharge from their containers and the application of mechanical pressure. Hair styling mousses, which rely upon the release of propellants to foam, are generally intended to be applied to wet or damp hair following shampooing and towel drying, although they may also be applied to dried hair.

The present invention solves this problem by providing an article which comprises a single phase, clear to the eye, aqueous composition which is to be discharged from an air-powered, non-aerosol pump and spray dispenser to form a foam-forming mousse, which composition comprises a clear solution of a monocationic quaternary surfactant salt, a hair holding polymeric material, and the remainder water, as well as the article comprising said dispenser and said composition. Optional ingredients may include a polycationic polymer, a voluminizing agent, a UV protectant, and a water soluble or miscible hair conditioning agent.

It is, therefore, an object of the present invention to provide a clear, mousse or quick-breaking foam composition, which is intended for use in environmentally friendly, non-aerosol, air-powered, pump and spray dispensers.

### SUMMARY OF THE INVENTION

This and other objects are realized by the present invention, one embodiment of which relates to the combination of an air-powered, non-aerosol pump and spray dispenser in which is packaged a composition that, upon discharge from the dispenser is in the form of a aqueous, clear to the eye, hair styling mousse, said composition comprising:
(i) 0.5-10% of a film-forming resin;
(ii) 0.1-10 of a monocationic quaternary surfactant capable of foaming in water:
(iii) 0-0.5% of a polycationic polymer;
(iii) 0-2% of a water soluble or miscible hair-conditioning agent;
(iv) 0-10% of a lower (C₁ to C₄) alkanol:
(v) 0-15% of at least one sunscreen agent;
(vi) miscellaneous adjuvants; and
(vii) the remainder water.

### DETAILED DESCRIPTION OF THE INVENTION

The present invention is predicated on the discovery that compositions that generate cosmetically acceptable styling mousses from environmentally friendly, non-aerosol (propellant-free), air-powered, pump and spray dispensers can be obtained by formulations that comprise: monocationic surfactants, film-forming resins, such as PVP/VA copolymers; and water. Such compositions can further contain high charge density cationic polymers, sunscreen agents, and water soluble hair conditioning agents such as silicones, aminosilicones, etc.

The general formula for the clear, mousse-forming solutions of the present invention is as follows:

| Ingredients | % By Weight |
|---|---|
| Film forming resin | 0.5-10 |
| Monocationic surfactant | 0.1-10 |
| Water soluble or miscible conditioner | 0-2 |
| Polycationic polymer | 0-0.5 |
| Lower (C₁ to C₄) Alcohol | 0-10 |
| Adjuvants | As needed |
| Deionized Water | Balance |

All percentages herein are by weight unless otherwise specified.

The preferred pump and spray, non-aerosol, air-powered dispenser of the present invention is made by Airspray International of Beverwijk, Netherlands and Pompano Beach, Florida, USA. It is said to have a unique tilt valve actuator, ad is designed to be an alternative to conventional gas-propelled aerosols. Generally, the dispenser containing the mousse-forming compositions disclosed herein will be pumped by the consumer about 10 times to generate sufficient air therewithin to permit the composition to foam upon its discharge from the dispenser. The air is the only propellant used in these articles. As the volume of the compositions is reduced by use, consumers will have to pump additional air into the dispenser to ensure optimum mousse formation.

The preferred film-forming polymer, which helps provide foaming of the mousse upon discharge from the dispenser and also forms a film coating on the hair to provide styling control, is a copolymer of vinyl acetate and vinylpyrrolidone monomers. It has the CTFA designation of PVP/VA Copolymers, and is commercially supplied by BASF as Luviskol 64. It contains 60% PVP and 40% VA and is supplied as PVP/VA-Water 50-50. Another useful polymer is the terpolymer of vinylcaprolactam, vinylpyrrolidone, and dimethylaminoethylmethacrylate. The polymer is generally present at levels of from about 0.5-10%, and preferably from about 2-10% , and most preferably from about 3-8%. At above 10%, the foaming quality of the composition may be lowered, and at below 0.5%, the styling effect of the product may not be noticeable.

The preferred monocationic quaternary salt, which also helps provide foaming of the mousse upon discharge from the dispenser and must be one that foams in water, is a quaternary ammonium compound having the following formula: 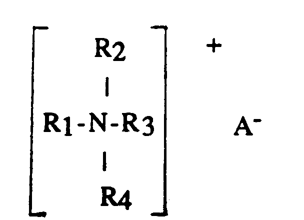
wherein R₁₋₃ are alkyl groups having from 1 to 4 carbon atoms, wherein R₄ is an alkyl group having from 8 to 16 carbon atoms, and wherein A is an anion. The preferred material has the CTFA designation of Cetrimonium Chloride (R₁₋₃ are each methyl groups, R₄ is an alkyl group having 16 carbon atoms, and A is Cl) and is commercially supplied by Berol Nobel as Querton 16C129 and contains 29% actives. This material must be present for foaming to take place upon release of the valve and dispensing of the mousse-forming composition. It also provides a conditioning effect, and it is generally present at levels of from about 0.1-10%, and preferably from about 0.2-0.5% , and most preferably from about 3-8%. At too high a level, the foaming quality of the composition may occur at the hair surface.

### Additional Optional Ingredients (Adjuvants)

Any polycationic polymer having hair conditioning properties may also be employed in the practice of the invention. These materials appear to increase hair volume (i.e. serve as "voluminizing agents"), impact on the creaminess of the composition, and fix on the negative sites on damaged hair shafts. Suitable such polycationic polymers include copolymers of methylvinylimidazolium chloride chloride and vinylpyrrolidone. A particularly suitable such polymer is BASF's LUVIQUAT FC 550 (CTFA designation: Polyquaternium 16; average molecular weight = 80,000), but Polyquaternium 11 (the quaternary ammonium polymer formed by the reaction of dimethyl sulfate and a copolymer of vinylpyrrolidone and dimethyl aminoethylmethacrylate) and Polyquaternium 24 (a polymeric quaternary ammonium salt of hydroxyethylcellulose reacted with a lauryl dimethyl ammonium substituted epoxide) also gave satisfactory results. This material may be present at a level of up to about 0.5%, with from about 0.02-0.3% being the preferred range. It will be understood that mixtures of suitable polycationic polymers may also be employed.

As a water soluble or miscible hair conditioning agent, the material having the CTFA designation Dimethicone Copolyol is preferred. It is a polymer of dimethylsiloxane with polyoxyethylene and/or polyoxypropylene side chains, and it is sold under various trade names including Dow-Corning 193 Surfactant. It acts to condition the hair and make it softer, as well as to prevent tangling during combing or brushing. Other water soluble or miscible silicones, including aminosilicones, should be useful, as long as they are sufficiently soluble in the aqueous medium to ensure that there is no precipitation, haziness, or formation of a second phase. This ingredient may be present at a level of up to about 0.5%, with from about 0.03-0.3% being the preferred range.

The mousse-forming compositions of the present invention may additionally comprise a lower (C₁-C₄) alkanol, such as methanol, ethanol or isopropyl alcohol, or a mixture thereof. While it can serve as a carrier or a diluent for the active ingredients of the composition, its primary function is to "dry" the mousse. While its presence is not necessary in the compositions of the present invention, it is desirable. This ingredient may be present at a level of up to about 10%, with from about 3-9% being the preferred range.

The mousse-forming compositions of the present invention may additionally include sunscreen agents (UV protectants) which protect the hair by blocking or reducing absorption of harmful UV radiation. This serves to inhibit bleaching of the hair. Preferred is the material having the CTFA designation Benzophenone-2. It is also known as 2,2',4,4'-Tetrahydroxybenzophenone and is sold by BASF under the trade name Uvinal D-50. The sunscreen agents used in the compositions of the present invention must be compatible with the water or water-alcohol base. Thus, it must be water miscible or water soluble, and it must not affect the clarity of the mousse-forming composition. Suitable sunscreen materials are disclosed in U.S. 4,567,038, the teachings of which are incorporated herein by reference.

The compositions of the invention can optionally contain dyes, perfumes, antioxidants, antimicrobials, preservatives, viscosity modifiers, and other auxiliary materials which are conventionally used in cosmetic compositions. For example, honey, D-Panthenol, and hydrolyzed collagen or animal protein may be used to provide additional conditioning, softness, body, gloss, etc.

The compositions should have a pH of from about 4.0 to about 5.0. Depending upon the other ingredients which may be selected for use in the composition, adjustment of pH to within this range with conventional pH adjustment agents (e.g., citric acid, sodium hydroxide) may be necessary.

The invention is illustrated by the following non-limiting example, and the following procedure was employed to formulate that composition. All percentages of components expressed herein, unless other indicated, are by weight, based on the weight of the composition in which the component is present.

The required amount of deionized water is added to a main mixing tank. The dye solution(s) are next added. The PVP/VA powder resin is next added and mixed well until homogeneous. The following raw materials, are then added in the in the following order: Cetrimonium Chloride; Polyquaternium 16; Dimethicone Copolyol; D-Panthenol; Honey; and the Hydrolyzed Collagen. If necessary, the D-Panthenol may be heated slightly to 40° C. to allow easier handling. In addition, care should be taken to ensure homogenization following addition of each ingredient. In a second tank, the requisite amount of ethanol is added, to which the Benzophenone 2 is added. This must be mixed well to ensure the dissolution of the UV absorbing material. Any fragrance is then added, and the mixture is transferred to the water phase. The product is then stirred until the product is homogenized and is in the form of a clear solution.

Following the above procedure, the composition set forth below was formulated. It should be understood that this specific example is illustrative of the nature of the mousse-forming composition of the present invention, but that the invention is not limited thereto.

| EXAMPLE | |
|---|---|
| Ingredients | % By Weight |
| Ethanol anhydrous, denatured | 7.00 |
| PVP/VA Copolymer (Luviskol 64 Powder-BASF 40% A.I.) | 6.00 |
| Dimethicone Copolyol (Surfactant 193-Dow Corning) | 0.10 |
| Cetrimonium Cl (Querton 16C129-Berol-Nobel 29% A.I.) | 1.00 |
| D-Panthenol | 0.10 |
| Honey | 0.10 |
| Hydrolyzed Collagen (Nutrilan 150-Henkel) | 0.25 |
| Polyquaternium 16 (Luviquat FC 550-BASF 40% A.I.) | 0.20 |
| Perfume | 0.10 |
| Color | 0.15 |
| Benzophenone 2 (Uvinal D50-BASF) | 0.10 |
| Deionized Water | Balance |
| Final pH= 4.5 | |

The efficacy of the above formulation was evaluated in a blind test run and compared to a conventional aerosol hair mousse, L'Oreal's "Studio Line."

A half head test was conducted by a hairdresser who used a conventional shampoo (an Aloe Vera variant containing 10% sodium lauryl ether sulfate and 3% betaine) to shampoo the hair of 26 female subjects. The L'Oreal aerosol "Studio Line" mousse was applied to half of each subject's head and the formulation of the Example was applied to the other half of each subject's head. First, the hairdresser evaluated each of the mousses as it was dispensed from the package. The results of the evaluation are shown in Section A of the Table. The mousse products were then applied to wet hair, and the hairdresser then evaluated the 4 characteristics which are shown in Section B of the Table. Following this, the hairdresser used a hair dryer to dry the hair of each subject and then compared the features of the dried hair, the results of which comparison are shown in Section C of the Table. The foregoing evaluations were based on a questionnaire with a score of 1-9 being given for each category, with 1 being the least and 9 being the best for each such category. In this connection, it is pointed out that hair mousse products are generally intended for use following shampooing and towel drying of the hair, but before blow drying with a hair dryer, to permit styling of the hair. Thus, how such products prevent or reduce hair tangling and promote easy combability of the hair is a prime indicator of their effectiveness. Other factors, such as adding volume to and conditioning the hair are also important. Of course, mousses can (and frequently are) used on dry hair for hair styling purposes.

**Table**

| | Studio Line Mean Score | Example Mean Score | Statistical Significance |
|---|---|---|---|
| | | | |

| A. Mousse Appln | | | |
|---|---|---|---|
| Aeration | 7.88 | 5.04 | 0.10 |
| Easy To Spread | 3.00 | 5.62 | 0.10 |
| Foam Stability | 7.92 | 5.19 | 0.10 |
| Foam Texture | 6.96 | 5.15 | 0.10 |

| B. Wet Hair | | | |
|---|---|---|---|
| Easy to Comb | 6.50 | 8.00 | 0.10 |
| Stickiness | 7.46 | 8.00 | 0.10 |
| Greasy | 8.00 | 8.00 | |
| Drying Time | 2.85 | 5.19 | 0.10 |

| C. Dry Hair | | | |
|---|---|---|---|
| Stiffness | 6.00 | 7.96 | 0.10 |
| Greasy | 8.00 | 8.00 | |
| Shining Hair | 2.15 | 2.15 | |
| Volume | 6.27 | 7.73 | 0.10 |
| Holding | 7.04 | 5.04 | 0.10 |

These test results indicate that the formula of the Example was significantly better than the L'Oreal Studio line with respect to the foam being easier to spread, wet hair being easier to comb, less sticky and drying in a shorter time than the L'Oreal product. The formula of the Example was also found to result in softer (less stiff) dry hair and a higher volume of hair. On the other hand, the formula of the Example was also found to give a somewhat less dense, less stable, less creamy, and less holding foam than the L'Oreal product. While this result is to be expected when comparing an aerosol mousse to a non-aerosol mousse product, nevertheless, the test results clearly demonstrate that the formula of the Example provides an acceptable, commercial quality mousse composition despite the fact that it is propellant-free.

Generally speaking, the present invention is directed to a single phase aqueous composition which is to be discharged from an air-powered, non-aerosol pump and spray dispenser to form a mousse, which composition comprises a clear solution of a monocationic quaternary surfactant salt, a hair holding, film forming polymeric material, and the remainder water, as well as the article comprising said dispenser and said composition. Optional ingredients may include polycationic polymers, UV protectants or sunscreen agents, preservatives, and a water soluble hair conditioning agent.

It is understood that the foregoing detailed description is given merely by way of illustration without departing from the spirit of the invention. It will be clear to those skilled in the art that modifications and/or variations of the disclosed compositions may be made without departing from the scope of the invention disclosed herein.

## Claims

1. A clear, single phase, aqueous composition which is to be discharged from an air-powered, non-aerosol, propellant-free, pump and spray dispenser to form a mousse, which composition comprises a monocationic quaternary surfactant salt that foams in water, a hair holding polymeric material, and the remainder water.

2. The composition of claim 1 which further comprises a polycationic polymer.

3. The composition according to claim 2 wherein said polycationic polymer is selected from the group consisting of Polyquaternium 16, Polyquaternium 11, Polyquaternium 24, and mixtures thereof.

4. The composition according to claim 3 wherein said polycationic polymer is Polyquaternium 16.

5. The composition according to claim 3 wherein the amount of said polycationic polymer ranges from about 0 to about 0.5%.

6. The composition of claim 1 which further comprises a water soluble or miscible hair conditioning agent.

7. The composition of claim 6 wherein said hair conditioning agent is selected from the group consisting of silicones, aminosilicones, and mixtures thereof.

8. The composition according to claim 7 wherein said silicone is dimethicone copolyol.

9. The composition according to claim 6 wherein the amount of said hair conditioning agent ranges from about 0 to about 0.5%.

10. The composition according to claim 1 wherein said monocationic surfactant is cetyl trimethyl ammonium chloride.

11. The composition according to claim 1 wherein the amount of said monocationic surfactant ranges from about 0.1 to about 10%.

12. The composition according to claim 1 wherein said film-forming polymer is selected from the group consisting of: PVP/VA Copolymer and the terpolymer of vinylcaprolactam, vinylpyrrolidone, and dimethylamino ethylmethacrylate.

13. The composition according to claim 12 wherein said film-forming polymer is PVP/VA Copolymer.

14. The composition according to claim 12 wherein the amount of said film-forming polymer ranges from about 0.5 to about 10% and more preferably from about 2-10% , and most preferably from about 3-8%.

15. The composition of claim 1 which further comprises a lower (C₁-C₄) alkanol.

16. The composition of claim 15 wherein said lower alkanol is selected from the group consisting of methanol, ethanol, isopropyl alcohol and mixtures thereof.

17. The composition of claim 15 wherein the amount of said lower alkanol ranges from about 0 to about 10% and more preferably from about 3-9%.

18. The composition according to claim 1 additionally containing mousse adjuvants selected from the group consisting of water miscible or water soluble sunscreen agents or UV protectants, dyes, perfumes, antioxidants, antimicrobials, preservatives, viscosity modifiers, honey, D-Panthenol, hydrolyzed collagen or animal protein and mixtures thereof.

19. The composition according to claim 18 wherein said sunscreen agent is Benzophenone-2.

20. An article comprising an air-powered, non-aerosol pump ad spray dispenser and a single phase, clear, aqueous composition which is housed within and designed to be discharged from said dispenser to form a foaming mousse, said composition comprising a monocationic quaternary salt, a hair holding polymeric material, and the remainder water.

21. The combination of an air-powered, non-aerosol, propellant-free pump and spray dispenser and a composition which is packaged therein, wherein said composition, upon discharge from said dispenser, is in the form of an aqueous, clear to the eye, hair styling mousse, and wherein said composition comprises:
(i) 0.5-10% of a film-forming resin;
(ii) 0.1-10 of a monocationic quaternary surfactant capable of foaming in water:
(iii) 0-0.5% of a polycationic polymer;
(iii) 0-2% of a water soluble or miscible hair-conditioning agent;
(iv) 0-10% of a lower (C₁ to C₄) alkanol;
(v) 0-15% of at least one sunscreen agent;
(vi) miscellaneous mousse adjuvants; and
(vii) the remainder water.

22. The combination of claim 21 wherein said polycationic polymer is selected from the group consisting of Polyquaternium 16, Polyquaternium 11, Polyquaternium 24, and mixtures thereof.

23. The combination of claim 22 wherein said polycationic polymer is Polyquaternium 16.

24. The combination of claim 23 wherein the amount of said polycationic polymer ranges from about 0 to about 0.5%.

25. The combination of claim 21 wherein said hair conditioning agent is selected from the group consisting of silicones, aminosilicones and mixtures thereof.

26. The combination according to claim 25 wherein said silicone is dimethicone copolyol.

27. The combination according to claim 26 wherein the amount of said hair conditioning agent ranges from about 0 to about 0.5%.

28. The combination according to claim 21 wherein said monocationic surfactant is cetyl trimethyl ammonium chloride.

29. The combination according to claim 28 wherein the amount of said monocationic surfactant ranges from about 0.1 to about 10%.

30. The combination according to claim 21 wherein said film-forming polymer is selected from the group consisting of: PVP/VA Copolymer and the terpolymer of vinylcaprolactam, vinylpyrrolidone, and dimethylamino ethylmethacrylate.

31. The combination according to claim 30 wherein said film-forming polymer is PVP/VA Copolymer.

32. The combination according to claim 31 wherein the amount of said film-forming polymer ranges from about 0.5 to about 10% and more preferably from about 2-10% , and most preferably from about 3-8%.

33. The combination of claim 21 which further comprises a lower (C₁-C₄) alkanol.

34. The combination of claim 33 wherein said lower alkanol is selected from the group consisting of methanol, ethanol, isopropyl alcohol and mixtures thereof.

35. The combination of claim 34 wherein the amount of said lower alkanol ranges from about 0 to about 10% and more preferably from about 3-9%.

36. The combination according to claim 21 additionally containing mousse adjuvants selected from the group consisting of water miscible or water soluble sunscreen agents or UV protectants, dyes, perfumes, antioxidants, antimicrobials, preservatives, viscosity modifiers, honey, D-Pathenol, hydrolyzed collagen or animal protein and mixtures thereof.

37. The combination according to claim 36 wherein said sunscreen agent is Benzophenone-2.
